# EUROPEAN PATENT APPLICATION

(11) **EP 4 793 947 A1**
(43) Date of publication of application: **19.08.2026**
(21) Application number: 25305202.1
(22) Date of filing: 14.02.2025
(51) Int. Cl.: G10L 25/51, A61B 5/00, G08B 21/02, G06N 3/08

(54) **TRAINING OF A MODEL TO CLASSIFY AUDIO FRAMES INTO AN EXPANDABLE SET OF SOUND CLASSES**

(71) Applicant: ORIKIO, 29200 Brest (FR)
(72) Inventor: DEBUNNE, Gilles, 29200 Brest (FR); PALLUAU, Nathan, 29200 Brest (FR); GARET, Gaëlle, 29200 Brest (FR); CREFF, Jordan, 29200 Brest (FR); DEMANGE, Guillaume, 29200 Brest (FR)
(74) Representative: Plasseraud IP

(57) **Abstract**

The invention relates to the detection of sound classes in audio frames. More specifically, the invention relates to the training of a second sound model when additional classes are added to an initial set of sound classes that has been used to train a first sound model.

## Description

### Technical Field

This disclosure pertains to the field of audio processing. More specifically, the disclosure pertains to the field of training models dedicated to the classification of audio frames.

### Background Art

Remote monitoring of an environment relates to remotely verifying what is happening in a given environment. For example, remote monitoring solutions are used in places where fragile people are found, to automatically raise alarms when fragile people are in danger. Remote monitoring typically consists in using sensors to perform measurements in the environment and analyzing the measurements in order to detect the state of the environment, thereby detecting if there is need for intervention in the environment. The remote monitoring thus provides, through the automatic analysis of sensor measurements, an automatic monitoring of a large number of different environments by a limited number of human operators. Human intervention in a given environment can be performed only if the remote monitoring indicated that such an intervention may be useful.

One typical example of an environment that needs to be remotely monitored is the rooms of retirement homes. Indeed, aged people may be subject to various hazards such as falls for example. Some aged people may also perform dangerous actions such as, for example, leaving their rooms unaccompanied. It is usually not possible in a retirement home to provide a human supervision of each room. Therefore, remote monitoring systems allow a central supervision of a large number of rooms by a limited number of operators, or, alternatively, improve the level of quality of care or assistance services provided by operators. As early as an alert is raised in one room, a human intervention can be provided to the room to verify if there indeed is a hazard and provide assistance if needed to the occupant of the room.

Other examples of remote monitoring of an environment are for example the remote monitoring of hospital rooms, medical homes or homes for disabled people. In this case also, human supervision of each room is in practice not possible. Thus, remote supervision of the room allows detecting for example if a patient is in danger in one of the rooms, so as to trigger a human intervention. Additionally, it can preserve patients' sleep quality by, for example, informing nurses that it is not the right time to enter the room if it is not necessary.

Many other examples of environments may be remotely monitored. For example, a human may be monitored remotely by wearing sensors, a portion of a street may be remotely monitored to detect violent events, etc.

Remote monitoring may be performed by different means. For example, it is most of the time performed by placing cameras in the environment, and automatically analyzing the images captured by the cameras.

However, remote monitoring based on cameras has important drawbacks. The most important drawback is that the cameras only capture images in a limited field of view. It is extremely difficult to obtain a complete representation of an environment, for example a room, based only on cameras. Even when putting a large number of cameras in the room (which would render the remote monitoring system more expensive and resource consuming), unmonitored areas may still be present.

Other drawbacks of remote monitoring based on cameras include the resource consumption. Indeed, video analysis is known to be very computationally expensive. Furthermore, when the captured videos are analyzed in a central server, the bandwidth requirements for transmitting the videos are important, especially in case of high-resolution videos. Resource issues are even more important when a plurality of cameras is located in the remotely controlled environments.

Using microphones instead of cameras alleviates these drawbacks. Indeed, a microphone placed in a room is able to capture all the sounds that are produced in the room. Thus, all a room, or a large portion of an outdoor environment, can be monitored using a single microphone. Furthermore, audio data is usually much smaller than video data, thereby significantly limiting the computing and bandwidth resources needed to process and analyze the microphone data compared to video.

Remote monitoring based upon microphones thus relies upon an analysis of the sound captured by the microphones. Such processing may include using a model to classify audio frames into one or more sound classes. Machine learning models (more simply called "models") are efficient for analyzing sound captured by a microphone because they can be trained on labeled datasets to recognize and classify various sound patterns. By learning from examples, these models can generalize their knowledge to accurately predict sound classes in new, unseen audio data. This capability allows for precise detection and classification of sounds, which is essential for applications such as remote monitoring and alert systems.

Models are usually trained from labeled training datasets. The sound detection training datasets for models are usually obtained by collecting a large number of audio frames and manually labeling them within a set of sound classes. This process involves playing the audio frames through a loudspeaker and having labelers listen to them and assign appropriate labels. These labeled audio frames are then used to create training samples, which are used to train the models to recognize and classify audio frames based on the labeled data.

In order to obtain an accurate classification, it is necessary to obtain a large training dataset. Obtaining an accurate classification therefore requires a large amount of time and effort from the labelers. The training of the model is typically continuously improved by progressively adding new training samples to the sound detection training dataset, then re-training the model.

In some instances, it may be beneficial to add additional sound classes to an initial set of sound classes. Such cases for example arises:
- when one aims at using the model in new environments, where sounds that were not present in the initial set of sound classes can be identified ,
- if additional sound classes, not previously foreseen, need to be detected.

However, such additional sound classes added to an existing, and possibly large training dataset, do not benefit from previously labeled training samples. This means that the new sound classes lack the extensive labeling that the original classes have. This results in an imbalance in the training data, where the new classes have significantly fewer labeled examples, potentially leading to less accurate detection and classification of these new sound classes by the model. Stated otherwise, adding additional sound classes then labeling additional training samples using the updated set of classes comprising the additional sound classes would result in a training set with rare training samples labelled using the additional sound classes. The original classes would therefore have significantly higher weight in the training than the additional classes. The training would therefore "erase" the additional sound classes, that would hardly be taken into account.

One solution to tackle this problem would be to entirely re-labeling the sound detection training dataset. Entirely re-labeling the sound detection training dataset would however be long and cumbersome because it involves manually reviewing and labeling each audio frame again, which is a time-consuming and labor-intensive process. Given the potentially large size of the dataset, this task would require a significant amount of effort and resources, making it impractical to perform frequently or for large datasets. Furthermore, such a solution would significantly delay the completion of the training of the model, and thus significantly delay the ability of the model to be deployed to effectively detect the additional sound classes.

There is therefore a need of a method and system to add additional sound classes to a sound detection training dataset of model, and provide an accurate detection of the additional sound classes in a short time with limited labeling effort.

### Summary

This disclosure improves the situation.

It is proposed a computer-implemented method comprising:
- obtaining a first trained sound model configured to detect in audio frames one or more sound classes belonging to an initial set of sound classes ;
- receiving from one or more microphones a plurality of audio frames ;
- for each audio frame of said plurality of audio frames:
   ∘ causing one or more loudspeaker of one or more Man Machine Interface to play said audio frame ;
   ∘ obtaining one or more labels belonging to an extended set of sound classes comprising the classes of the initial set of sound classes, and at least one additional sound class ;
   ∘ enriching a sound detection training dataset of a second sound model with said audio frame associated to said one or more labels ;
- using said sound detection training dataset of the second sound model to train said second sound model to detect in audio frames one or more sounds classes belonging to said extended set of sound classes.

By "model", or "machine learning model", we designate a model, or function, that can be trained on a training dataset, in order to generalize its learning on unseen data. The training phase of the model allows determining parameters of the model or function that best fit the training set, while the inference phase makes use of the trained model to perform predictions on unseen data.

The sound models used here are usually trained using supervised learning (also called supervised machine learning), which means that they are trained based on inputs associated to desired outputs called labels. Models that can be trained using supervised learning encompass, for example, models such as artificial neural networks, decision trees, support vector machines or random forests. Models may typically be classifiers that aim at classifying input data in certain classes. In such case, the labels that are provided for the training can be the classes identified by a labeler in the training samples.

By "sound model", we designate a model that aims at detecting sounds classes from audio frames.

The first and second sound models may detect the sound classes in different ways. For example, each of the sound models may perform one of the following tasks:
- a classification task: in such case, the model can be qualified as *"a classifier"* and is configured to detect the presence or absence of each class. In such case, a model may for example output a vector indicating, for each class, the presence or absence of the class, and/or a confidence index of the presence or absence of the class ,
- a regression task: in such case, the model can be configured to predict, for each class, a ratio of positive labels associated to the class (e.g a ratio of predictions of the presence of the class among a plurality of predictions).

By "label belonging to a sound class", we designate a label indicating the absence or presence of a sound class in an audio frame (or equivalently the classification or not of the audio frame in the sound class).

By "label belonging to a set of sound classes", we designate a label indicating the absence or presence of one of the sound classes of the set in an audio frame (or equivalently the classification or not of the audio frame in a sound class of the set).

The first trained sound model has been previously trained to detect in audio frames sounds belonging to the initial set of sound classes.

Meanwhile, the second trained sound model is trained to detect in audio frames sounds belonging to the extended set of sound classes including at least one additional sound class. The second sound model thus allows detecting the additional sound classes using a limited number of labels.

Thus, the detection of sound classes in audio frames can benefit from the first trained model that has been trained with an extensive dataset on the initial set of sound classes, but also from the second trained model that has been trained using the at least one additional class.

The method thus allows benefiting from an accurate detection in the initial set of sound classes, while being able to detect sound in the at least one additional classes using limited number of additional labels.

For example, when a new additional sound class is considered, it can be detected quickly while benefiting from a precise prediction for all the previously known audio classes.

In another aspect, it is proposed a computer-implemented method comprising
- receiving from one or more microphone an audio frame ;
- detecting in said audio frame one or more sound classes belonging to an extended set of sound classes, using one or more of:
   o a second sound model trained using at least a part of a method as defined here ;
   o a first sound model re-trained using at least a part of a method as defined here.

Making use of one or more of the second sound model, and the first sound model allows benefiting from the predictions of a model that is trained to accurately detect the at least one additional sound classes.

In another aspect, it is proposed a computer software comprising instructions to implement at least a part of a method as defined here when the software is executed by a processor.

In another aspect, it is proposed a computer-readable non-transient recording medium on which a software is registered to implement at least a part of a method as defined here when the software is executed by a processor.

In another aspect, it is proposed a computer system comprising:
- one or more Man Machine Interface comprising one or more loudspeaker ;
- one or more processing unit configured to:
   ∘ obtain a first trained sound model configured to detect in audio frames one or more sound classes belonging to an initial set of sound classes ;
   ∘ receive from one or more microphone a plurality of audio frames ;
   ∘ for each audio frame of said plurality of audio frames:
      ▪ cause one or more loudspeaker of one or more Man Machine Interface to play said audio frame ;
      ▪ obtain one or more labels belonging to an extended set of sound classes comprising the classes of the initial set of sound classes, and at least one additional sound class ;
      ▪ enrich a sound detection training dataset of a second sound model with said audio frame associated to said one or more labels ;
   ∘ use said sound detection training dataset of the second model to train said second sound model to detect in audio frames one or more sounds classes belonging to said extended set.

In another aspect, it is proposed a computing system comprising:
- one or more microphone
- one or more first computing device ;
wherein said one or more first computing device is configured to:
- receive from said one or more microphone an audio frame ;
- detect in said audio frame one or more sound classes belonging to an extended set of sound classes, using one or more of:
   ∘ a second model trained using at least a part of a method as defined here ;
   ∘ a first sound model re-trained using at least a part of a method as defined here.

The following features, can be optionally implemented, separately or in combination one with the others:

In an embodiment, the method further comprises:
- retrieving a sound detection training dataset of the first sound model, said sound detection training dataset of the first sound model comprising a plurality of initial samples, each initial sample comprising an audio frame associated with one or more labels belonging to the initial set of sound classes ;
- enriching the sound detection training dataset of the first sound model, with labels belonging to said at least one additional sound class, said enriching comprising, for each initial sample:
   ∘ using the second sound model to detect in the audio frame of the initial sample the presence or absence of said at least one additional sound class ;
   ∘ enriching the one or more labels associated to the audio frame of the initial sample with the detection of presence or absence of said at least one additional sound class, so that the audio frame of the initial sample is associated with labels of the extended set of sound classes;
- re-training the first sound model using the enriched sound detection training dataset of the first sound model.

The predictions performed by the second sound model allow predicting the presence or absence of the at least one additional sound classes in the samples of the sound detection training dataset of the first model, that are initially labelled using the initial set of sound classes.

Thus, all the samples of the sound detection training dataset of the first sound model can be enriched to be associated with labels of the extended set of sound classes rather than labels of the initial set of sound classes only.

This operation can be done automatically, without requiring manually labelling the dataset of the first model, which can be large.

Thus, the first sound model can be re-trained to be able to detect all the classes of the extended set of sound classes instead of only the classes of the initial set of sound classes, without requiring manual labelling of a large dataset of training samples.

In an embodiment, the method further comprises:
- retrieving a test dataset of the first sound model, said test dataset comprising a plurality of test samples, each test sample of the test dataset comprising an audio frame associated to one or more labels belonging to the initial set of sound classes ;
- enriching said test dataset with labels belonging to the at least one additional sound class received through said one or more Man Machine Interface (MMI2);
- using said enriched test dataset to verify the training of the re-training of the first sound detection model.

This allows enriching the test dataset with manual labels belonging to the additional sound classes, so that all the samples of the test dataset have manually entered labels for all the classes of the extended set of sound classes.

Thus, even if the re-training of the first sound model is performed using artificially generated labels for the additional sound classes, the verification of the training is performed using a test dataset that comprises manual labels for all the sound classes, thereby ensuring an adequate test of the training .

In an embodiment, enriching said test dataset with labels belonging to the at least one additional sound class comprises, for each test sample of the test dataset, or a subset of the test dataset :
- causing one or more loudspeaker of said one or more Man Machine Interface to play the audio frame of the test sample ;
- receiving, through said one or more Man Machine Interface, one or more labels belonging to the at least one additional sound class ;
- enriching the test sample with said one or more labels belonging to the at least one additional sound class.

It is worth noting that the sound detection test datasets typically have a much smaller size than the sound detection training datasets.

Thus, a limited amount of manual labels allow training the first model using the extended set of sound classes, while ensuring that the test of the training is performed using manual labels.

In an embodiment, enriching the sound detection training dataset of the first sound model further comprises, adding training samples from the sound detection training datasetof the second sound model to said sound detection training dataset of the first sound model

As the sound detection training dataset of the second sound model comprises training samples that have been manually labelled in the extended set of sound classes, the training samples of the sound detection training dataset of the second sound model learning can be integrated in the sound detection training dataset of the first sound model, so as to benefit from additional manually labeled training samples and improve the training of the first sound model.

In an embodiment, enriching the sound detection training dataset of the first sound model further comprises:
- receiving from said one or more microphone a plurality of subsequent audio frames ;
- for each subsequent audio frame of said plurality of subsequent audio frames:
   ∘ causing said one or more loudspeaker of said one or more Man Machine Interface to play said subsequent audio frame ;
   ∘ receiving, through said one or more Man Machine Interface, one or more labels belonging to the extended set of sound classes;
   ∘ enriching the sound detection training dataset of the first sound model with said subsequent audio frame associated to said one or more labels .

Once the sound detection training dataset of the first sound model comprises samples labelled in the extended set of sound classes, it can be enriched with subsequent received training samples labelled in the extended set of sound classes, so that the first model can be trained with the highest possible number of training samples.

In an embodiment, the first and the second sound models are convolutional neural networks.

Convolutional neural networks provide an effective solution to classify sound in audio frames.

In an embodiment, the first and the second sound models are neural networks having a same architecture.

By "architecture of a neural network", we refer to the structure of the neural network, which includes the arrangement and connection of its layers and neurons. A typical neural network consists of an input layer, one or more hidden layers, and an output layer. Each layer is composed of nodes or neurons, which are interconnected by weighted edges. The input layer receives the data, the hidden layers process the data through weighted connections and activation functions, and the output layer produces the final prediction or classification.

Two neural networks that have, or share, the same architecture, are therefore characterized by the same layers, neurons, transfer functions and weighted edges.

The first and second models are both neural networks having the same architecture, so that only the sound detection training dataset used to trained them differ. Thus, upon the training, the parameters of the networks, including hyperparameters, automatically adjust to the differences in the datasets Thus, a consistent behavior of the first and second models can be obtained.

In an embodiment:
- a loss function used for training at least one of the first sound model and the second sound model comprises a weighted sum of prediction losses of each class of the extended set of sound classes ;
- the weights of the prediction loss of each class of the extended set of sound classes in said weighted sum is a decreasing function of the number of occurrences of the sound classes in the sound detection training dataset of said at least one of the first and the second sound model.

A decreasing function is a type of mathematical function where, as the input value increases, the output value either remains the same or decreases. In other words, for any two input values x₁ and x₂ such that x₁<x₂, the function f satisfies the condition f(x₁) ≥ f(x₂). The decreasing function may be either:
- a strictly decreasing function, where, for any two input values x₁ and x₂ such that x₁<x₂, the function f satisfies the condition f(x₁) > f(x₂) ;
- a non-increasing function, where there may be some values x₁ and x₂ such that x₁<x₂ and f(x₁) = f(x₂).

This allows to provide more weight to the errors on labels of rare or underrepresented classes in the training, so that each sound class is equally taken into account, even if a limited number of labels belong to some sound classes in the sound detection training dataset.

In an embodiment, said extended set of sound classes comprises at least one super-class that encompasses a plurality of classes of the extended set of sound classes.

This allows benefiting from an efficient learning of the super-class, while providing the option of labelling of a super-class corresponding to a general type of sounds, rather than a labelling in a specific class, for when the detection of a specific class in an audio frame is not possible.

In an embodiment, at least one among the first sound model and the second sound model is configured to perform a regression task to predict, for each sound class, a ratio of received labels that indicate the presence or the absence of the sound class, respectively state class.

When a plurality of labels are obtained for a same audio frame (for example if a plurality of labels and/or pseudo-labels are received respectively from a plurality of labelers and/or pre-trained machine models for a sample), it is common that some labels differ (e.g some labelers / pre-trained model indicate that a given sound or state class is present from a given audio frame, while other indicate that the sound or state class is absent). It is thus possible to determine, for each class, a ratio of positive labels and negative labels (for example, 80% of labels indicate that the sound class "squeak" is present in a given audio frame).

A ratio of received labels that indicates the presence or the absence of a sound class thus refers to a quantitative measure that represents the proportion of labels indicating the presence of a specific class relative to the total number of labels received for that class. This ratio is typically expressed as a fraction or percentage and is used to assess the likelihood that one or more labelers or pre-trained models indicate the class as present in the audio frame.

For example, if an audio frame is labeled by multiple labelers, and 8 out of 10 labelers indicate the presence of the sound class "dog bark," the ratio of received labels indicating the presence of the "dog bark" sound class would be 0.8 (or 80%). Conversely, the ratio indicating the absence of the "dog bark" sound class would be 0.2 (or 20%).

Predicting a ratio of received labels that indicate the presence or the absence of the sound class rather than a classification (presence / absence of a class, or index of confidence) allows providing more accurate results, because it allows preserving the complete information received in the creation of the sound detection training dataset. This is even more the case for the predictions of sound classes, because the ratios obtained as output of the sound model can be directly provided as input to the state model.

For example, if 4 out of 5 labelers indicated that an audio frame contains the sound class "squeak", training the sound model to predict that 80% of labels indicated the presence of the class retains more information that simply predicting that the class was present. The accuracy of the predictions is therefore increased with the same received labels.

In an embodiment, an audio frame at a time frame is sent by said one or more microphone only upon a condition relative to a sound level from said audio frame.

Avoiding sending audio frames when no noticeable sound level has been recorded significantly reduces the amount of data sent by the microphones.

### Brief Description of Drawings

Other features, details and advantages will be shown in the following detailed description and on the figures, on which:
**Fig. 1**

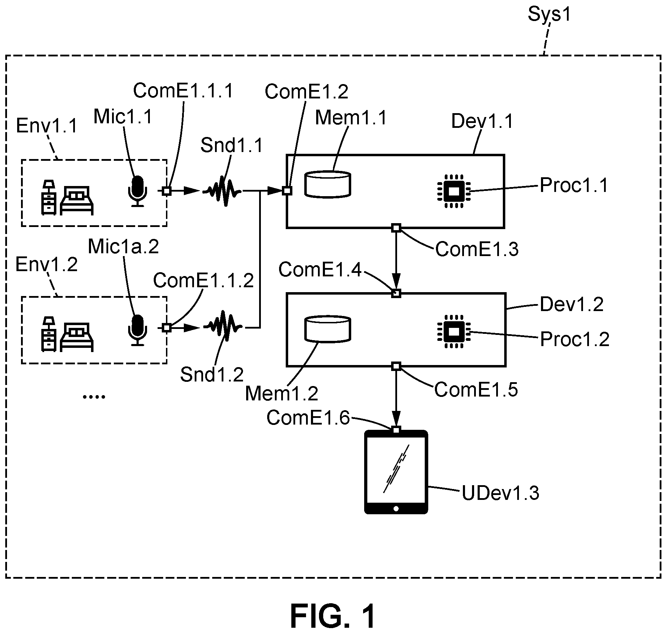

   [Fig. 1] is an example of a computing system to remotely monitor one or more environments that makes use of a model trained according to an embodiment.
**Fig. 2**

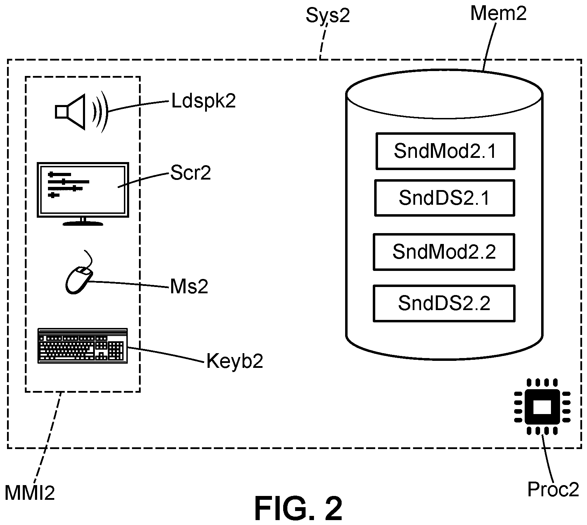

   [Fig. 2] is an example of a computing system to train a plurality of sound models.
**Fig. 3**

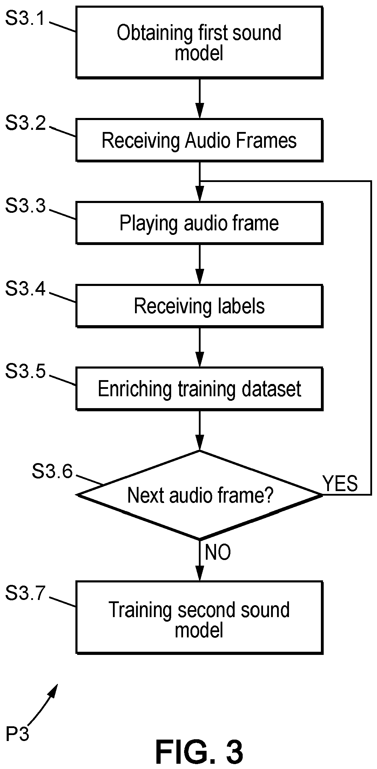

   [Fig. 3] is an example of a method to train a second model to detect sound classes in audio frames in an embodiment.
**Fig. 4**

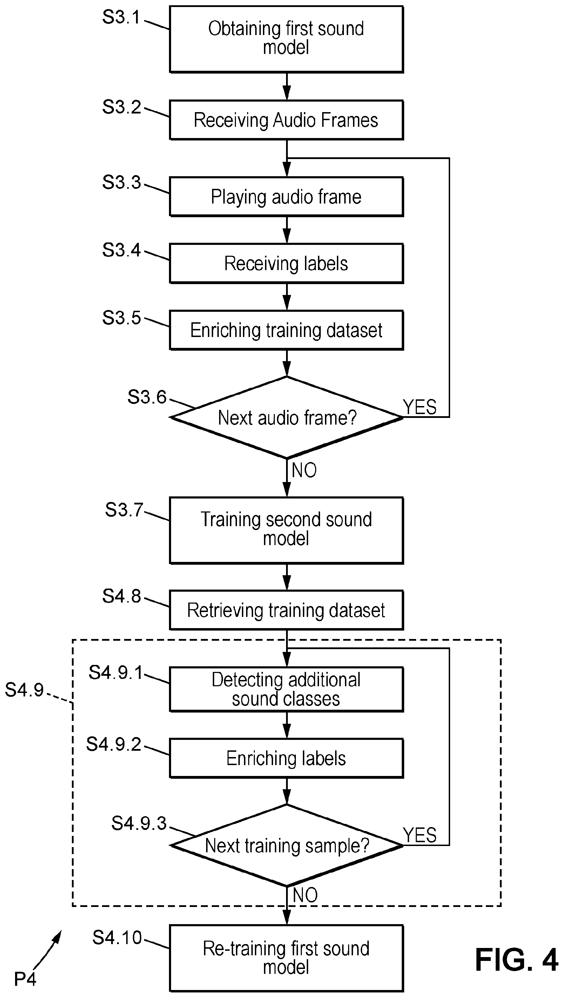

   [Fig. 4] is an example of a method to train a first and a second model to detect sound classes in audio frames in an embodiment.
**Fig. 5**

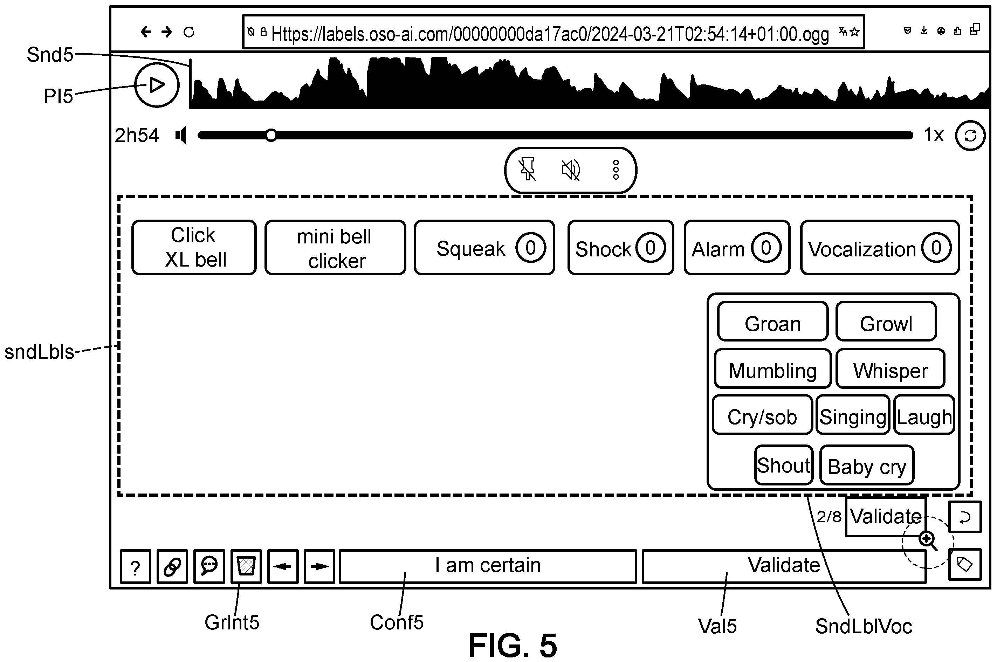

   [Fig. 5] is an example of a graphical interface to allow a labeler to associate sound labels to a sound frame according to an embodiment.
**Fig. 6**

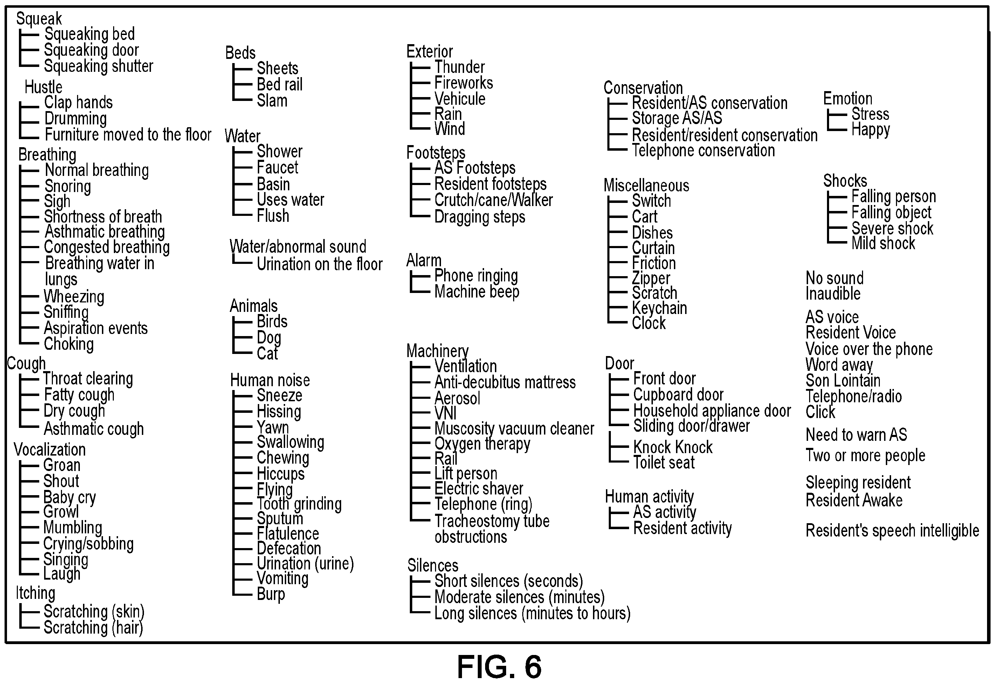

   [Fig. 6] is an example of sound classes according to an embodiment.
**Fig. 7**

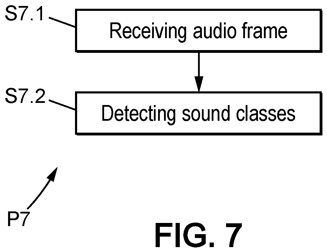

   [Fig. 7] is a method of detection of sound classes in an audio frame according to an embodiment.

### Description of Embodiments

It is now referred to figure 1.

Figure 1 is an example of a computing system Sys1 to remotely monitor one or more environments Env1.1, Env1 .2 according to an embodiment.

The system Sys1 is an example of a computing system to remotely monitor one or more environments that makes use of a model trained according to an embodiment. It is worth noting that figure 1 is provided by means of non-limitative example only of a system that makes use of a model for detection of sound classes in audio frames trained according to an embodiment of the invention. The models according to the invention may also be used in many other systems, for many different purposes. More generally, the sound models trained according to an embodiment of the invention may be used within any kind of system that requires the classification of sound frames captured by microphones, and wherein new sound classes may be added.

By "an environment", we designate a fixed or mobile location that is affected by human activity. For example, the environment may be an environment of a person such as for example a room where the person lives, a hospital bedroom where the person sleeps or even the person itself. The environment may also be a place that is affected by human activity without being tied to one specific person. For example, such an environment may be a classroom, a part of a street, a child playground, etc. Non limitative examples of environments comprise:
- a room :
   ∘ a bedroom of a retirement home ;
   ∘ a bedroom of a hospital ;
   ∘ a bedroom in a house ;
   ∘ a classroom ;
   ∘ etc.
- a portion of an exterior place:
   ∘ a playground ;
   ∘ a part of a street;
   ∘ etc.
- a vehicle, or portion of a vehicle:
   ∘ the interior of a car ;
   ∘ a wagon of a train ;
   ∘ a cockpit of a plane ;
   ∘ etc.
- a person or an animal:
   ∘ an old person wearing a microphone to detect hazards ;
   ∘ a sick person ;
   ∘ a child ;
   ∘ an animal ;
   ∘ etc.
- etc.

In the example of figure 1, the system Sys1 is a remote monitoring system of one or more retirement homes. In the example of figure 1, the monitored environments are thus two bedrooms of a retirement home, noted respectively Env1.1 and Env1.2. Thus, the system Sys1 aims at automatically detecting, from the sound captured by the microphones, if something unusual happened in the bedrooms of the residents of the retirement homes.

To this effect, one or more microphones are placed in each of the environments. In the example of figure 1, one microphone Mic1.1 is placed in the room Env1.1, and one microphone Mic1.2 is placed in the room Env1.2.

The microphones are configured to capture sounds in their respective environments and send the audio frames comprising the captured sound to a first computing device Dev1.1. In the example of figure 1, the first microphone Mic1.1 sends a first audio frame Snd1.1, and the second microphone Mic1.2 sends a second audio frame Snd1.2. The communications are performed between a communication endpoint and ComE1.1.1 of the first microphone Mic1.1, a communication endpoint ComE1.1.2 of the second microphone Mic1.2, and a communication endpoint ComE1.2 of one or more first computing device Dev1.1. The communication may be performed using any suitable communication means including wired or wireless telecommunication network. For example, Ethernet networks, 4G/5G networks, optical fiber networks, etc. may be used.

It is worth noting that the term "microphone" may refer to a device that comprises a microphone and a communication endpoint. For example, the term "microphone" may refer to a housing that comprises a microphone, a communication endpoint, and one or more processing units to process the signals from the microphone to create the audio frames to send via the communication endpoint.

The one or more microphones may be associated to unique identifiers (which may be for example a unique identification number of the microphone, an indication of the location of the microphone, etc.), so that the environment to which each microphone belongs to can be traced during all the process of analyzing audio frames, then raising alerts, so that an alert can be associated with the right environment.

The audio frames may be sent in different audio formats. For example, raw formats of compressed formats may be used. Different sound depths (8 bits, 16bits, 24bits, 32 bits for instance), or sound formats (mono, stereo for instance) may be used. Each microphone may integrate, or somehow associate, its unique identifier to the audio frames, so that the detection can be associated to a particular microphone and environment.

Each frame may be associated with a timestamp, which may for example be a frame identifier or a time of recording.

In a number of embodiments, the microphones may send the audio frames only upon a condition relative to a sound level in said audio frame. For example, a microphone may send an audio frame:
- only if the average sound intensity in the audio frame is above a predefined threshold ; or
- only if a maximum sound intensity in the audio frame is above a predefined threshold ;
- if a temporally close peak of sound intensity is identified near the audio frame. For example, the audio frames may be sent up to 3 seconds before and 7seconds after the sound intensity is equal to or higher than a predefined threshold, for example expressed in dB ;
- the sound intensity may be considered for a single microphone, or a plurality of microphones. In the latter case, the sound intensity may correspond to different combinations of the sound signals outputted by the microphones (e.g average of the sound signal or energy of the sound signals of a plurality of microphones, difference between the sound signals of two microphones or the energy of the sound signals of two microphones, etc.)
- etc.

Stated otherwise, the microphones may send only the frames for which a sufficiently high sound level is detected, because the audio frames with only very low sound will not be considered as carrying information. Thus, the amount of data sent by the microphones is reduced and the system consumes a lower amount of bandwidth, without reducing the accuracy of the detection.

In addition, in order to ensure that a minimum number of audio frames are transmitted, an audio frame can be transmitted at regular intervals even in absence of noticeable sounds. For example, an audio frame can be transmitted every 10 minutes even in absence of noticeable sound. In an embodiment, a longer frame (for example an audio frame of 2 minutes instead of 10 seconds) can also be sent from time to time, for example every 7 hours.

When an audio frame is not transmitted by a microphone to the device Dev1.1, the absence of transmission may be signaled in different ways. In an embodiment, no data is transmitted. The detection of the absence of reception of audio frame at a given timestamp then indicates that the frame has not been transmitted. Alternatively, an explicit signaling indicating the absence of transmission may be sent.

The one or more first computing device Dev.1.1 comprises one or more memories Mem1.1, and one or more processing units Proc1.1.

"Processing unit" means an electronic component capable of performing electronic or computer calculations for a function. A processing unit can designate any type of processor or electronic component capable of performing digital calculations. For example, a processing unit can be an integrated circuit, an ASIC (from the English acronym "Application-Specific Integrated Circuit", literally in French "integrated circuit specific to an application", a microcontroller or microcontroller unit (MCU), a microprocessor, a Digital Signal Processor (DSP), a processor, a Graphical Processing Unit (GPU). A processing unit according to the invention is not limited to a particular type of calculation architecture. For example, a processor can implement a Harvard or Von Neumann type architecture.

"Memory" means a digital electronic device used to store data. Different types of memories can be used in the invention, such as read only memory, random access memory, volatile memory or flash memory. A device according to the invention can be equipped with one or more non-volatile memories which can be of different types such as mass memories, flash memory, read only memories or SSD memories. A device according to the invention may also comprise one or more random access memories such as RAM, DRAM, SRAM, DPRREAM, VRAM, eDRAM or 1T-SRAM.

The one or more processing units Proc1.1 are configured to detect states of the environments based upon the received audio frames. To this effect, the one or more processing unit Proc1.1 is configured to analyze the audio frames, and deduce the states of the environments. For example, the first processing unit may detect the states of the rooms of the retirement home, to determine for example if the resident of the room is fallen, left the room unaccompanied, if the TV is playing, etc. When the audio frames are associated to a unique identifier of a microphone, the state can be associated to the corresponding environment.

By "state of an environment", we designate an indication of what happens in the environment. For example, a state of an environment may represent :
- an action that is performed by one or more persons in an environment, such as "running", "laughing", "crying", etc.
- an indication of the state of the elements of the environments, for example "TV playing", "music playing", "squeaky bed", etc. ;
- an indication of an event that just occurred in the environment, such as for example "person falling", "person leaving the room", etc.
- an indication of the presence or absence of person or animals ;
- when the environment is a person or an animal, a state of the person or the animal, such as for example "has fallen", "long cry", "repeated screaming", "distress", etc.
- etc.

To this effect, the states of the environments can be classified into one or more classes, and some of the classes may be representative of an alert state (for example a fall or an old person leaving his/her room unaccompanied). When a class representative of an alert state is detected, an alert can be raised to the personnel of the retirement home.

In the example of figure 1, the detection of the states of the environment relies upon a classification of the sounds of the environment, wherein a model determines the sound classes that are present within each audio frame received from the microphones. For example, the identification of cough, booms, sound of TV, etc. provides an insight about the states of the environments, for example the actions happening in each environment, or the health condition of the resident of a room of the retirement home.

In the example of figure 1, in order to raise an alert:
- the alert is sent by a communication endpoint ComE1.3 of the one or more computing device to a communication endpoint of one or more second computing device ;
- the one or more second computing device determines a user device UDev1.3 to which the alert is to be transmitted, and transmit, from a communication endpoint ComE1.5 of the one or more second computing device, the alert to a communication endpoint ComE1.6 of the user device UDev1.3 where the alert is to be dispatched.

To this effect, the one or more second computing device is equipped with one or more processing unit Proc1.2 and one or more memory Mem1.2. The one or more processing unit Proc1.2 is configured to determine to which user device the alert is to be dispatched depending upon the environment that is concerned by the alert and/or the alert type. For example, the one or more processing unit Proc1.2 may determine, based on the alert type and an identifier of the microphone from which the audio frames that caused the alert were sent.

For example, the user devices UDev1.3 may be tablets, smartphones or other user devices of the personnel of the retirement homes where the rooms Env1.1 and Env1.2 are found. Thus, the one or more computing device may determine to which retirement home, and optionally to which personal, the alert is to be sent. For example, certain rooms may be associated to a specific personal that is in charge of the room. The personnel to which the alert is sent may also depend upon the type of alert. Some alerts may be for example medical emergency, that are sent directly to a medic, some other may be sent to caregivers, etc.

Once an alert is received by a user device UDev1.3, the alert may be rendered to the user of the user device. For example, a visual indication may be displayed of the screen of the user device, indicating the type of alert, as well as an indication of the environment, such as for example the room number, or the name of the resident of the room. Other alert means may be rendered to the user, such as for example beeps, sound alerts, etc.

Thus, the system Sys1 allows detecting different states and events of the rooms based upon the sounds detected in the audio frames received from the microphones, and dispatch alerts as early as possible so that the personal can perform interventions as early as possible. As the alerts are based upon the sound data that is captured by the microphones, everything that happens in the room can be monitored. The system Sys1 therefore provides an effective means of remotely monitoring a large number of rooms of retirement homes and perform interventions as early as possible when aged people require assistance.

In some embodiments, some classes that are not representative of alert classes can also be transmitted. Thus, the personal of the retirement homes can benefit from a remote monitoring that is not limited to alerts. For example, they can know which residents are singing, watching TV, etc.

The example of figure 1 is provided by means of non limitative example only of a computing system of the disclosure. The disclosure is however not limited to this system. For example:
- the number of rooms that are monitored may be different from 2. For example, a single room or more than two rooms may be monitored. The monitored room may belong to one or a plurality of different retirement homes ;
- the monitored environments may be different from a retirement homes. For example:
   ∘ fixed environments such as hospital rooms, classrooms, children playground, portions of streets may be monitored ;
   ∘ mobile environments such as a vehicle, a portion of a vehicle of a person can be monitored. In such cases, position information can be sent, for example by the microphones, in addition to the audio frames or in audio frames so that, when an alert is raised, an intervention can be performed at the right location (for example the current location of the vehicle or the person).
- a monitored environment may be an environment of a person, for example a bedroom of that person in a retirement home or a hospital, or an environment that is not specifically tied to a person, such as for example a classroom or a portion of street ;
- more than one microphone can be placed in a given environment ;
- the computing system may be a computing system that is internal to a single structure (for example, a computing system of a single retirement home), or a computer system that encompasses a plurality of structures (for example, a plurality of different retirement homes). In the former case, the computing devices may be located in the single structure, and local communication networks such as Wi-Fi or Ethernet may be used. In the latter case, the computing devices may be external to the structures. The computing devices may be for example servers of a remote monitoring service provider, and global communication networks such as optical fiber or 4G/5G networks may be used for the communication between the microphones and the computing devices ;
- even if a single computing device Dev1.1 is represented is figure 1, a plurality of first computing devices may be used, for example a server farm ;
- even if a single computing device Dev1.2 is represented is figure 1, a plurality of second computing devices may be used, for example a server farm ;
- even if a single user device UDev1.3 is represented is figure 1, a plurality of user devices may be used ;
- even if a single processing unit Proc1.1 is represented is figure 1, a plurality of processing units may be used by the one or more first computing device Dev1.1, for example a plurality of cores of a multi-core processor, a plurality of processors, a plurality of processors spread across a plurality of computing device (in case distributed computing is used), etc.
- even if a single processing unit Proc1.2 is represented is figure 1, a plurality of processing units may be used by the one or more second computing device Dev1.2, for example a plurality of cores of a multi-core processor, a plurality of processors, a plurality of processors spread across a plurality of computing device (in case distributed computing is used), etc.
- the alerts that are raised are dependent upon the environment that is monitored. The dispatch of the alert through the one or more second computing devices and user devices is purely illustrative, and any suitable means of dispatching alerts can be used according to various embodiments of the invention ;
- the dispatch of alerts through the one or more second computing device Dev1.2, and the user device UDev3.1 is provided by means of purely illustrative example only. For example:
   ∘ the one or more first computing device Dev1.1 can directly transmit alerts to user devices ;
   ∘ the alerts can be transmitted to central service rather than user devices ;
   ∘ the monitoring of states is not necessarily done to raise alert, but may for example be done for obtaining statistics relative to the actions performed in the environment
   ∘ etc.

The system Sys1 is thus provided by means of illustrative example only of a computing system that makes use of a model trained according to an embodiment. It is apparent that the objective of the invention is to improve the training of sound models. The system Sys1 is thus provided as a mere example of a system that makes use of the sound classification. However, according to different embodiments of the invention, many other uses of the sound classifications may be foreseen. The many possible uses of sound classification for example comprise:
- Monitoring hospital rooms to detect patient distress or medical emergencies.
- Surveillance of public spaces, such as streets or parks, to detect violent events or accidents.
- Monitoring the well-being of individuals in their homes, such as detecting falls or unusual activities.
- Animal behavior monitoring, such as detecting distress calls or unusual sounds from pets or wildlife.
- Enhancing smart home systems by recognizing specific sounds, such as doorbells, alarms, or appliance noises.
- Improving security systems by detecting sounds indicative of break-ins or other security breaches ;
- Etc.

As already mentioned, the system Sys1 relies upon a previously trained model to detect sound classes within the audio frames received from the microphones. In some instances, it may be desirable to add new sound classes to detect. It is for example the case if one aims at detecting new types of alerts, or if the system Sys1 is used to monitor a new environment that requires the detection of other kinds of sound classes. In such cases, it is desirable to re-train the model in a reasonable time, while in the same time benefiting from the previous labels, and providing a balanced training between the original sound classes (which benefit from a large number of labels), and the newly added sound classes (which do not benefit at this stage from a large number of labels).

The same problem arises in any system that makes use of a previously trained model to detect sound classes within sound frames sent by one or more microphone.

It is now referred to figure 2.

Figure 2 is an example of a computing system to train a plurality of sound models.

The computing system Sys2 may be for example a personal computer, a tablet, a server or more generally any kind of computing system that has access to one or more Man Machine Interface. The computing system Sys2 thus comprises one or more Man Machine Interface MMI2, and one or more processing unit Proc2.

The Man Machine Interface may be at least partially located in the same computing device than the one or more processing unit Proc2 (which is for example the case of an internal loudspeaker or an internal microphone of a tablet).

The one or more Man Machine Interface MMI2 may be also be located outside the computing device where the one or more processing unit are located. This may be for example the case of a Man Machine Interface in a wired or wireless local connection with the computing device, such as for example a mouse, keyboard, or a microphone or loudspeakers connected through a wire or a Bluetooth connection to the computing device. This may also be the case for example if the interface with the user is performed in a local device, and the training in a distant computing device in connection with the local device.

The one or more Man Machine Interface MMI2 comprises one or more Loudspeakers Ldspk2. The one or more Loudspeakers Ldspk2 allow rendering audio frames to a user of the system sys2. The one or more Man Machine Interface MMI2 may further comprises other interfaces such as one or more screen Scr2 and/or one or more mouse Ms2 and or one or more keyboard Keyb2. Other types of Man Machine Interfaces, not represented in the figure 2, may also be considered. For example, a microphone may be used. It is apparent that the system Sys2 is not restricted to specific types of Man Machine Interfaces, provided that the user is able to listen to audio frames, and associate labels to the audio frames.

In general, the user can associate each audio frame to one or more sound labels belonging a set of sound classes. The sound labels represent the sounds that can be heard in the audio frames. For example, the sound classes may define different types of sounds such as noises, breathing, clapping, flush, dog, bird, cat, phone ring, sing, laugh, etc. ;

The provision by the user of sound labels in response to rendering audio frames on the loudspeakers allows enriching, on one or more memory Mem2 of the system Sys2, one or more sound detection training dataset such as for example the sound detection training dataset SndDS2.1 , SndDS2.2 where the audio frames are associated to sound labels.

The plurality of sound detection training dataset SndDS2.1, SndDS2.2 are respectively used to train one or more sound model (also called more shortly "sound models") SndMod2.1, SndMod2.2 to detect one or more sounds classes in audio frames. Lore specifically, the sound detection training dataset SndDS2.1 is used to train the first sound model SndMod2.1, and the sound detection training dataset SndDS2.2 is used to train the second sound model SndMod2.2. The sound detection training dataset SndDS2.1 is called "sound detection training dataset of the first sound model SndMod2.1", while the sound detection training dataset SndDS2.2 is called "sound detection training dataset of the first sound model SndMod2.2".

The sound models may for example be classifiers, i.e machine learning models that are configured to classify a sound into one or more sound classes.

The sound models SndMod2.1 and SndMod2.2 may thus be classifiers that are configured to output respectively a vector having one element for each class of a set of sound classes, wherein each element provides an indication of the detection of a class. The indication of the detection of a class may be for example a Boolean value indicating the detection or lack of detection of a class, a confidence value of the presence of a class, etc. Thus, no sound class, or more than one sound classes may be detected.

Alternatively, the sound models may perform a regression task to predict a ratio of labels indicating the presence or absence of each class. Such a label may for example be expressed as a percentage, or a natural number in an interval [0; 1]. In such case, the output of the sound models would be a vector having one element for each class of a set of sound classes, wherein each element provides a ratio of labels indicating the presence or absence of the detection of the class.

Of course, the first and second sound models may belong to different types of models. For example, the sound models may perform a regression task, or a classification task.

In particular the first sound model SndMod2.1 and the second sound model SndMod2.2 can be convolutional neural networks (CNNs), that are known to provide an effective solution to classify audio frames into sound classes.

In particular, the first sound model SndMod2.1 and the second sound model SndMod2.2 can share a same architecture, so that a consistent behavior between the first and second models SndMod2.1 and SndMod2.2 can be obtained.

For example, the first sound model SndMod2.1 and the second sound model SndMod2.2 can both be one of the versions of the "MobilNet" Convolutional Neural Network described by:
- Howard, A. G., Zhu, M., Chen, B., Kalenichenko, D., Wang, W., Weyand, T., ... & Adam, H. (2017). Mobilenets: Efficient convolutional neural networks for mobile vision applications. arXiv preprint arXiv:1704.04861. *;*
- Sandler, M., Howard, A., Zhu, M., Zhmoginov, A., & Chen, L. C. (2018). Mobilenetv2: Inverted residuals and linear bottlenecks. In Proceedings of the IEEE conference on computer vision and pattern recognition (pp. 4510-4520).. ;
- Howard, A., Sandler, M., Chu, G., Chen, L. C., Chen, B., Tan, M., ... & Adam, H. (2019). Searching for mobilenetv3. In Proceedings of the IEEE/CVF international conference on computer vision (pp. 1314-1324);

According to various embodiments of the invention, an audio frame may be input to the sound model in different ways:
- the waveform (e.g succession of values of audio samples) can be directly fed to the sound model, for example if the sound model is based on a CNN in 1D (e.g a CNN that takes as input data in 1 dimension such as a 1-column or 1-row vector, that may be a waveform for example). This solution preserves the whole content of the audio frame and may provide precise results, especially when a large training dataset has been built ;
- a spectrogram of the audio frame may be provided as input to the sound model. For example, a 2D-image of the spectrogram of the frame may be constructed, and provided as input to the sound model, for example if the sound model is a 2D CNN (e.g a CNN that takes as input data in 2 dimensions such as an image for example). This solution may provide satisfactory results, even when only a small training dataset can be used.

More generally, a sound model may take as input the audio frame itself, or an input derived from the audio frame, such as for example a frequency transform of the audio frame, or a spectrogram of the audio frame. The type of the input may also depend upon the inputs that are accepted by the sound model, especially in terms of number of dimensions.

The operations performed by the computing system Sys2 in order to train the models are described in more details with reference to figures 3 and 4.

It is now referred to figure 3.

Figure 3 is an example of a method to train a second model to detect sound classes in audio frames in an embodiment.

The method P3 aims at training a second sound models (such as SndMod2.2). The method P3 can for example be implemented by the computing system Sys2.

It is first worth noting that the second sound model may be trained for one or more environment. For example:
- a sound model may be specifically trained for a given environment (e.g a specific room, car, person, portion of street, etc.) ;
- a sound model may be specifically trained for a given group of environments. Such groups of environments may either relate to environments that belong to a same structure (for example all the rooms of a given retirement home, all the rooms of a given hospital etc) ;
- a sound model may be specifically trained for a given type of environments. A type of environments may refer to environments that share some degrees of similarity. For example, rooms of retirement homes in general, rooms of hospital in general, rooms that are near a street, etc.

As already explained, a sound model is a model that takes as input an audio frames and delivers as output a prediction of sound classes that are present from the audio frame (which is equivalent to classifying the audio frame into one or more sound classes).

The prediction of sound classes may for example take the form of a vector that comprises for each sound class an indication of presence or absence of the sound class (e.g Boolean value, confidence of presence, etc.), if the sound model is a classifier.

The prediction of sound classes may also in another example take the form of a vector that comprises for each sound class a ratio that indicates what would be the ratio of labels representative of presence or absence of the sound class among a plurality of labels received for the audio frame.

The method P3 is for example used when a first sound detection model SndMod2.1 is already trained, or will be trained using a first training set SndDS2.1 that uses an initial set of sound classes, and detections according to at least one additional sound class, not present in the initial set of sound classes, are required. Thus, detections using an extended set of sound classes comprising the initial set of sound classes, and the at least one additional sound class, are required.

Such situation arises for examples when an initial set of sound classes is used to detect sound classes in known environments, and:
- if one aims at using the model in new environments, where sounds that were not present in the initial set of sound classes can be identified ,
- if at least one additional sound classes, not previously foreseen, need to be detected. For example, an initial set of sound classes that comprises a class "wheelchair" can be enriched with sub-classes "motorized wheelchair", and "muscle powered wheelchair", if one considers that there is a need for distinguishing the detection of the sounds of different kinds of wheelchairs ;
- etc.

As explained above, simply adding training samples labelled using the extended set of sound classes to the first training set SndDS2.1, then re-training the first sound model SndMod2.1 would not provide accurate detection results, because the first training set would then be imbalanced between the classes of the initial set of sound classes that would benefit from a large number of training samples, and the at least one additional sound classes that would rely only on a limited number of training samples.

One objective of the training method P3 is therefore to ensure that the training is balanced between the initial and additional sound classes, and that a model can be trained rapidly with a limited labeling effort to detect sound classes using the extended set of sound classes.

The method P3 comprises a first step S3.1 of obtaining a first trained sound model, such as the model SndMod2.1 configured to detect in audio frames one or more sound classes belonging to an initial set of sound classes.

The first trained sound model SndMod2.1 can be obtained in different ways.

For example, the first trained sound model SndMod2.1 may be a pre-existing model that has already been trained on a dataset to recognize an initial set of sound classes.

The first trained sound model SndMod2.1 may also be obtained through a preliminary training step, for example using an interface such as the one represented in figure 5, or using steps similar to the steps S3.2 to S3.7 described below, but applied to the first sound model SndMod2.1, and the initial set of sound classes.

The computer-implemented method P3 comprises a second step S3.2 of receiving a plurality of audio frames (for example Snd1.1 and/or Snd1.2) from one or more microphone (for example Mic1.1 and/or Mic1.2).

In an embodiment, the microphones may be microphones located in one or more environment to monitor, or similar to other embodiments that will be monitored. This is for example the case if the audio frames are received from the microphones Mic1.1 and/or Mic1.2, respectively located in the environments Env1.1 and Env1.2.

The microphones from which the audio frames are received may for example depend upon the environments for which the training is performed. For example:
- if the training is performed for a single environment (for example Env1.1), only audio frames from the microphones in that environment may be received (for example Mic1.1) ;
- if the training is performed for a group of environments, only audio frames from the microphones in that group of environment may be received ;
- if the training is performed for a type of environments, only audio frames from microphones in environments belonging to this type may be received.

Then, the steps S3.3 to S3.6 are then executed for each of audio frame of the plurality of received audio frames.

The step S3.3 consists in causing one or more loudspeaker (such as the loudspeakers Ldspk2 for example) of one or more Man Machine Interface (such as the Man Machine Interface MMI2 for example) to play the audio frame.

The step S3.4 consists in obtaining one or more sound label belonging to a set of sound classes.

For example, the one or more sound label may be received through the one or more Man Machine Interface, such as the interface MMI2. In this case, the one or more sound label can be provided by one or more labeler after having listened to the audio frame. For example, the one or more sound label may be received may be received through an interface such as the interface shown in figure 5.

Stated otherwise, the steps S3.3 and S3.4 consist in having a labeler listen to an audio frame, then indicate which sound classes of the extended set of sound class he/she has heard in the audio frame.

It is noteworthy that, as the labels are received in the extended set of sound classes, the labeler has the opportunity to label the audio frames into the at least one additional sound class.

Alternatively, some of the sound labels may be provided at step S3.4 by a pre-trained model. For example, such a model may be a specialized model trained to detect a specific sound class, a general model that has been previously trained. For example, the sound labels may be provided by a very large model to train a lightweight model. Thus, accurate labels may be provided for the training, but the model that runs in inference phase has a low resource consumption.

In substance, the steps S3.3 and S3.4 thus allow one or more user of system such as the system Sys2 for example to label the training examples, by listening the audio frames and associating the audio frames to sound classes. For the sake of simplicity, the figure 3 will be discussed with reference to "a user" that performs the labelling. However, the method is also applicable to a plurality of users that perform the labelling, for example a plurality of users that label different audio frames. The user of the labeling system may be called "labeler" in the subsequent parts of the description.

Of course, the sound classes may be dependent on the environment. For example, the state classes may be different if the environment is a room of a retirement home or a child playground.

The labelling at steps S3.4 may be performed in different ways. For example, the user may click of buttons representative of each class that he/she has detected, type the noun of the classes, etc. The figure 5 provides an example of an interface for labelling the audio frames into sound classes in an embodiment of the invention.

The step S3.5 consists in enriching a sound detection training dataset (for example the sound detection training dataset SndDS2.2) of the second sound model (for example SndMod2.2) with the audio frame associated to said one or more sound label belonging to the extended set of sound classes.

The enriching consists in adding a training example comprising the audio frame and the associated one or more sound label. Thus, the sound detection training dataset can be enriched with new training examples thanks to the labels provided by the labeler or pretrained model. The enriching can be performed in different ways.

In a number of embodiments, a single label is received for each audio frame. Stated otherwise, the audio frame is labeled by a single labeler or pseudo-labeled by a single pre-trained model. In such case, the training sample will simply associate the audio frame to the received label (indication or presence or absence) of each class.

In other embodiments, a plurality of labels or pseudo-labels are received, from different labelers and/or pre-trained models, for each audio frame and sound class. In such case, the enriching comprises adding the label received, and, when all the expected labels are received, combining them into a single label.

For example, a plurality of labelers, pre-trained models or a combination thereof may label or pseudo-label each audio frame. In such case, a training sample may be provided in different ways. For example:
- If the sound model is a classifier, the plurality of labels may be combined into a single label of presence of absence of the classes. For example, a ratio of labels of presence can be calculated and compared to a threshold. For example, if a threshold of 50% is used, and 5 labelers label a frame, a sound class can be considered as present if at least 3 labelers among the 5 (60% > 50%) indicate that the sound class is present ;
- If the sound model performs a regression task, a ratio is calculated to indicate the ratio or percentage of labelers that considered the class as present or absent. For example, if 3 out of 5 labelers indicated that a given sound class is present, a ratio of 0.6 or 60% can be associated to the class.

After step S3.5, in an embodiment a step S3.6 consists in verifying if a subsequent received audio frame to label exists. If yes, the steps S3.3 to S3.5 are repeated for the subsequent audio frame to label. Otherwise, if all the received audio frames have already been processed, the sound detection training dataset has been completely enriched, and the step S3.7 is executed.

The step S3.7 consists in using the second sound detection training dataset SndDS2.2 of the second sound model SndMod2.2 to train the second sound model SndMod2.2 to detect one or more sounds classes from audio frames. Thus, the second sound model SndMod2.2 can be trained to infer, from the audio frames, the sound labels as defined in the training samples.

Thus, at the output of the method P3, two sound models are obtained:
- the first sound model SndMod2.1 benefits from a large history of labeling on the initial set of sound classes ;
- the second sound model SndMod2.2 benefits from a labeling using the extended set of sound classes, and thus the at least one addition sound class. Meanwhile, the training of the second sound model SndMod2.2 is balanced, since the extended set of class is used for the labeling of all the training samples of the training set of the second sound model SndMod2.2.

Thus, predictions of sound classes can benefit from accurate predictions for all the classes, because one can make use of both the first sound model SndMod2.1 (notably for predictions in the initial set of sound classes), and the second sound model SndMod2.2 (notably for predictions in at least one additional sound class)

According to various embodiments of the invention, the sound detection training dataset SndDS2.2 of the second sound model SndMod2.2 can be either completely created during by the method P3, an existing training dataset can be enriched, or a new training dataset can be derived from an existing one.

In some embodiments, a sound detection training dataset is formed only from the training samples created with the audio frames received at step S3.2 Thus, a complete creation of a sound detection training dataset is performed.

In other examples, a pre-existing training dataset is enriched with new training samples. For example:
- a generic dataset can be enriched with training samples from a specific environment to create a sound detection training dataset adapted to this specific environment. For example, a generic dataset of sound and states from rooms of retirement homes can be enriched with training samples from a given room of a given retirement home, or all the rooms of a retirement homes, to create a dataset specifically adapted to a given room or retirement home ;
- a pre-exiting training dataset associated to a given environment, group of environments or type of environments is enriched with new training samples from that environment, group of environments or type of environments. Thus, the sound detection training dataset can be expanded and/or take into account new sound or state classes ;
- a sub-set of the sound detection training dataset SndDS2.1 of the first sound model SndMod2.1 may be used as pre-existing dataset. This allows obtaining a larger training dataset SndDS2.2 for the second sound model SndMod2.2, while obtaining a fairly balanced training, if the training samples that were labelled using the extended set of sound classes represent a significant number compared to the training samples that were labelled using the initial set of sound classes.

The training at step S3.7 can be performed using any known method for training a model. For example, if a models relies on an artificial neural network, the training may be performed using gradient descent methods. For example, one or more of the following below may be performed for the trainings:
- a plurality of iterations of optimizing parameters of the of the state and / or sound models using for example a gradient descent (for example a stochastic gradient descent such as described by Kingma, D. P., & Ba, J. (2014). Adam: A method for stochastic optimization. arXiv preprint arXiv:1412.6980.) in order to optimize a loss function such as for example a Binary Cross Entropy "BCE" or Kullback Leibler "KL" divergence loss function. The loss function may for example be applied to a binary classification ;
- a plurality of optimization of hyperparameters of the state and / or sound models, for example a Bayesian optimization using Parzen Tree Estimators ;
- etc.

In cases where the training is performed to minimize a loss function, as the training is performed in a plurality of classes of the extended set of class, each class is associated to a prediction loss, and the loss function to minimize relies on a sum (or average) of the prediction losses of each class of the extended set of sound classes.

However, the number of occurrences of the sound classes of the extended set of sound classes in the sound detection training dataset may be very different. Stated otherwise, some of the sound classes may be labelled many times, while other may be labeled a limited number of times. When the differences of the number of occurrences are too high between the most represented and the least represented classes, the classes may be unbalanced, and the training may take too much into account the most represented classes, because the high number of occurrences of the labels of those classes may influence too much the global loss function.

In order to avoid this issue,
- the loss function used for training at least one of the first and the second sound model may comprise a weighted sum of prediction losses of each class of the extended set of sound classes ;
- and the weight of the prediction loss of each class of the extended set of sound classes in said weighted sum may be a decreasing function of the number of occurrences of the sound classes in the sound detection training dataset.

Stated otherwise, different weights may be assigned to each sound class, and the weight of a class may be higher when the numbers of labels of this class is low, in order to balance the contribution of each sound class to the loss function.

For example, the weight assigned to each class may be inversely proportional to the number of labels of this class in the sound detection training dataset (or, equivalently, to the proportion of labels that relate to this class), so that each sound class provides the same contribution to the loss function.

Although the steps of the method P3 have been described with reference to the sound detection training datasets SndDS2.2 of the second sound model SndMod2.2, similar steps can be used to create other datasets relative to the second sound model SndMod2.2, such as for example a validation dataset and/or a test dataset of the second sound model SndMod2.2.

The orders of the steps of the method P3 in the figure 3 are provided by means of non-limitative example. In particular, the steps S3.3 and S3.5 are not necessarily perform sequentially, and may be performed in parallel for all the initial training samples, without executing the step S3.6.

Even though the figure 3 has been discussed with respect to the first sound detection model SndMod2.1, and the second sound detection model SndMod2.2 represented in figure 2, the method P3 is generally applicable to any first and a second sound models. The same remark is also applicable to embodiments discussed with figure 4.

Is it now referred to figure 4.

Figure 4 is an example of a method P4 to train a first and a second model to detect sound classes in audio frames in an embodiment.

The method P4 comprises all the steps of the method P3 discussed with respect to the figure 3.

In addition, the method P4 comprises the steps S4.8 to S4.10 discussed below.

The step S4.8 consists in retrieving the sound detection training dataset SndDS2.1 of the first sound model SndMod2.1.

The sound detection training dataset SndDS2.1 of the first sound model SndMod2.1 comprises a plurality of initial samples, and each initial sample comprises an audio frame associated with one or more labels belonging to the initial set of sound classes.

Stated otherwise, the step S4.8 consists in retrieving the sound detection training dataset SndDS2.1 that was used to train the first sound model SndMod2.1. As discussed below, the first sound model SndMod2.1 was trained to detect sound classes in the initial set of sound classes, that does not comprise the at least one additional sound class.

The step S4.8 of retrieving the sound detection training dataset SndDS2.1 can be performed in different ways according to the way the sound detection training dataset SndDS2.1 is stored. In general, the sound detection training dataset SndDS2.1 can be retrieved from one or more memory storage, such as the one or more memory storage Mem2, where it is stored. The dataset SndDS2.1 can thus be retrieved from a storage location where it is saved. This could be a database, a file system, or a cloud storage service where the dataset is maintained. The retrieval process involves accessing the storage medium and loading the dataset into the computing system for further processing.

If the dataset is stored in a database, a query can be executed to extract the relevant data. This query would specify the criteria for selecting the audio frames and their associated labels that form the sound detection training dataset SndDS2.1.

If the dataset is stored in files, such as CSV or JSON files, the files can be read and parsed to load the data into memory. This involves opening the files, reading the contents, and organizing the data into a format suitable for use in training the model.

In cases where the dataset is accessible through an API, a request can be made to the API to retrieve the dataset. The API would return the dataset in a structured format, which can then be used for training purposes.

The examples above are provided by means of non-limitative examples only of ways to retrieve the sound detection training dataset SndDS2.1. More generally any retrieval method that is relevant may be executed depending on the way the sound detection training dataset SndDS2.1 is stored.

The method P4 comprises, after the step S4.8, a step S4.9 of enriching the sound detection training dataset SndDS2.1 of the first sound model SndMod2.1., with labels belonging to said at least one additional sound class.

The step S4.9 therefore aims at adding to the initial samples of the sound detection training dataset SndDS2.1 of the first sound model SndMod2.1 labels representative of the at least one additional sound class, that was not used for initially labelling the initial samples.

To provide a (voluntarily simple) concrete example, let's imagine that the initial set of sound classes comprises, among the classes represented in figure 6, the sound classes "Birds" and "Dog" that represent respectively the sounds of birds and dogs. Thus, each training samples of the sound detection training dataset of the first sound model associates an audio frame to a label indicative of the presence of the "Birds" class, and a label representative of the "Dog" class.

Then an additional sound class "Cat" is added to represent the sounds of cats. Thus the extended set of sound classes comprises the classes "Birds", "Dog" and "Cat". The step S4.9 would in this case consist in adding to each initial sample of the sound detection training dataset SndDS2.1 a label representative of the presence or absence of the sound class "Cat", in addition to the labels representative of the presence or absence of the sound classes "Birds" and "Dog".

The step S4.9 comprises, for each initial sample
- a first sub-step S4.9.1 of using the second sound model to detect in the audio frame of the initial sample the presence or absence of the at least one additional sound class. In the simplified example discussed above, the sub-step S4.9.1 would for example consist in using the second sound model SndMod2.2 (which had been previously trained at step S3.7 and using the sound detection training dataset SndDS2.2 to detect all the sound classes of the extended set of sound classes, including the additional class "Cat") to detect the presence or absence of the additional class "Cat" in the audio frame of the initial sample belonging to the sound detection training dataset SndDS2.1 ;
- a second sub-step S4.9.2 of enriching the one or more labels associated to the audio frame of the initial sample with the detection of presence or absence of the at least one additional sound class, so that the audio frame of the initial sample is associated with labels of the extended set of sound classes. In the simplified example discussed above, the sub-step S4.9.2 would thus consist in adding to the labels associated with the audio frame of the initial sample a label indicative of the presence or absence of the additional sound class "Cat". At the output of the sub-step S4.9.2, the enriched initial sample thus associates the audio frame to labels representative of the presence or absence of the three sound classes "Birds", "Dog" and "Cat".

After the execution of the second sub-step S4.9.2, a sub-step S4.9.3 verifies if there is a further initial training sample to process. If the outcome of the verification is positive (e.g if there is at least one initial training sample that was not enriched with labels representative of the presence or absence of the at least one additional class), the method returns to the step S4.9.1 for a subsequent initial training sample.

Otherwise (if all the initial training sample have been enriched with labels representative of the presence or absence of the at least one additional class), all the enriched initial training samples of the enriched training dataset SndDS2.1 are now labelled using all the classes of the extended set of sound classes, including the at least one additional class. The method then processes to the step S4.10.

The step S4.10 consists in re-training the first model using the enriched sound detection training dataset of the first sound model. Started otherwise, the first model SndMod2.1 is re-trained using the enriched training SndDS2.1 to be able to detect all the sound classes of the extended set of sound classes: not only the initial sound classes, but also the at least one additional class.

Thus, at the output of step 4.10, the first model is able to detect all the sound classes of the extended set of sound classes (including the at least one additional sound classes). The first model thus benefits from a large training dataset. This operation does not require large amount of labeling efforts, since the labels representative of the at least one additional sound class are provided by the second model.

The step S4.9 of enriching the sound detection training dataset (SndDS2.1) of the first sound model may comprise additional sub-steps in addition to the sub-steps S4.9.1 to S4.9.3 to further increase the size of the sound detection training dataset.

For example, enriching the sound detection training dataset (SndDS2.1) of the first sound model may further comprise adding training samples from the sound detection training dataset SndDS2.2 of the second sound model to said sound detection training dataset SndDS2.1 of the first sound model.

In practice, this sub-step consists in duplicating all or a part of the training samples of the sound detection training dataset SndDS2.2 of the second sound model into the sound detection training dataset SndDS2.1 of the first sound model.

As the training samples of the sound detection training dataset SndDS2.2 of the second sound model SndMod2.2 comprise samples manually labelled in the extended set of sound classes, the duplication of all or a part of the training samples of the sound detection training dataset SndDS2.2 of the second sound model into the sound detection training dataset SndDS2.1 of the first sound model SndMod2.1 increases the size of the sound detection training dataset SndDS2.1 with high quality training samples, thereby improving the training of the first sound model SndMod2.1.

Another option for further enriching the sound detection training dataset consists in adding subsequent training samples (for example training samples comprising audio frames received or labeled after the training of the second sound model SndMod2.2), which are directly labeled using the extended set of sound classes.

To this end, the step S4.9 of enriching the sound detection training dataset (SndDS2.1) of the first sound model may further comprises:
- receiving from said one or more microphone a plurality of subsequent audio frames ;
- for each subsequent audio frame of said plurality of subsequent audio frames:
   o causing said one or more loudspeaker of one or more Man Machine Interface, such as the one or more Man Machine Interface MMI2, to play said subsequent audio frame ;
   o receiving, through said one or more Man Machine Interface, one or more labels belonging to the extended set of sound classes;
   o enriching the sound detection training dataset SndDS2.1 of the first sound model SndMod2.1 with said subsequent audio frame associated to said one or more labels.

Stated otherwise, the manual labelling steps are here directly applied to subsequent received audio frames using the extended set of sound classes, in order to enrich the sound detection training dataset SndDS2.1 of the first sound model SndMod2.1. The size of the sound detection training dataset SndDS2.1 is thus increased with high quality manually labeled training samples.

The orders of the steps of the method P4 in the figure 4 are provided by means of non-limitative example. In particular, the steps S4.9.1 and S4.9.2 are not necessarily perform sequentially, and may be performed in parallel for all the initial training samples, without executing the step S4.9.3.

The execution of the additional steps S4.8 to S4.10 of the method P4 (compared to the steps of the method P3) may be caused upon different conditions.

In general, training dataset SndDS2.2 of the second sound model SndMod2.2 may be continuously enriched, and the second sound model SndMod2.2 trained one or more times, until the re-training of the first sound model SndMod2.1 is triggered.

In an embodiment, a user may trigger the enrichment of the sound detection training dataset SndDS2.1 (steps S4.9.1 and S4.9.3), and the re-training of the first sound model SndMod2.1, for example when he/she considers that the training of the sound detection training dataset SndDS2.2 reached a sufficiently large size, and/or an enrichment of the sound detection training dataset SndDS2.1 is needed.

The execution of the steps S4.8 to S4.10 may also be triggered by one or more conditions, such as for example:
- a validation, by a user, that the training of the second sound model SndMod2.2 is satisfying, for example that the accuracy of the classification of the at least one additional classes ;
- the number of training samples of the sound detection training dataset SndDS2.2 is equal to or higher than a predefined threshold. This condition indicates that the size of the sound detection training dataset SndDS2.2 is considered as sufficiently large, so that the second sound model SndMod2.2 provides accurate predictions and can be used to enrich the sound detection training dataset SndDS2.1 ;
- a confidence score of classification (for example Softmax function, sigmoid output, etc.) of the second sound model SndMod2.2 is on average equal to or higher than a predefined threshold. This condition indicates that the second sound model SndMod2.2 is sufficiently confident in its own classifications, so that they can be considered as sufficiently reliable to enrich the sound detection training dataset SndDS2.1. The confidence score of classification may be considered for all the classes of the extended set of sound classes, or only the at least one additional class. The confidence score of classification can be considered on a plurality of samples, for example the average confidence scores of classification can be considered on a validation dataset of the second sound model SndMod2.2.

The conditions for triggering the enrichment of the of the sound detection training dataset SndDS2.1 (steps S4.9.1 and S4.9.3), and the re-training of the first sound model SndMod2.1 can also be combined. For example:
- a user can first validate that the training of the second sound model SndMod2.2 is satisfying, then conditions relative to the number of training samples, confidence score of classification, etc. are considered ;
- Cumulative conditions may be applied. For example, the enrichment of the of the sound detection training dataset SndDS2.1 (steps S4.9.1 and S4.9.3), and the re-training of the first sound model SndMod2 may be triggered if the number of training samples of the sound detection training dataset SndDS2.2 is equal to or higher than a predefined threshold AND a confidence score of classification (for example Softmax function, sigmoid output, etc.) of the second sound model SndMod2.2 is on average equal to or higher than a predefined threshold.

The conditions listed above for triggering the enrichment of the of the sound detection training dataset SndDS2.1 (steps S4.9.1 and S4.9.3), and the re-training of the first sound model SndMod2.1 are provided by means of non limitative example only. It is more generally apparent that any condition, either determined automatically or through user input, or combination of conditions indicating that the second sound model SndMod2.2 reached a sufficiently high accuracy for detection of the at least one additional sound class can be used.

In addition to the enrichment of the sound detection training dataset, the test dataset of the first sound model can thus be enriched with additional labels representative of the presence of absence of the at least one additional sound classes.

To this effect, a test dataset of the first sound model can first be retrieved. The test dataset comprises a plurality of test samples Each test sample of the test dataset comprises an audio frame is initially associated to one or more labels belonging to the initial set of sound classes, as the first sound model was previously trained to predict the sound classes of the initial set of sound classes.

The enriched test dataset can then be used to verify the training of the re-training of the first sound model, in order to test the predictions of the sound classes belonging to the at least one additional sound classes performed by the re-trained first sound model.

The test dataset of the first sound model SndMod2.1 can be enriched in the same way as the sound detection training dataset SndDS2.1, by using the second sound model SndMod2.2 to enrich the test samples of the test dataset with labels representative of the presence or absence of the at least one additional class.

Additionally or alternatively, some or all of the additional labels representative of the presence of absence of the at least one additional sound classes can be labels received through one or more Man Machine Interface such as the one or more Man Machine Interface MMI2 (e.g labels manually entered by a user).

Thus, when some or all of the additional labels representative of the presence of absence of the at least one additional sound classes can be labels received through one or more Man Machine Interface, the re-trained first sound model is performed using manually received labels relative to he at least one additional class.

Therefore, even if the labels relative tot he at least one additional class in the sound detection training dataset of first sound model SndMod2.1 have been generated automatically by the second sound model SndMod2.2, the test of the predictions of the at least one additional class by the first sound model SndMod2.1 will be performed using manually entered labels.

This allows avoiding that any inconsistencies in the predictions of the at least one additional sound class by the first sound model SndMod2.1 that would be due to the fact that the first sound model has been trained using pseudo-labels of the at least one additional class to remain unnoticed.

The enrichment of the test dataset of the first sound model with labels representative of the presence of absence of the at least one additional sound classes received through the one or more Man-Machine Interface MMI2 can be performed in different ways.

For example, test samples of a test dataset of the second sound model can be added to the test dataset of the first sound model.

As the second sound model has been built directly using the extended set of sound classes, the test dataset of the second sound model comprises labels relative to the at least one additional class that have been manually entered by a user through the one or more Man Machine Interface MMI2, which can thus be also added in the test dataset of the first sound model.

Additionally or alternatively, a user can add labels belonging to the at least one additional sound class (e.g labels representative of the presence or absence of the at least one additional sound class in an audio frame) in the existing test samples of the test dataset.

To this effect, enriching the test dataset of the first sound model with labels belonging to the at least one additional sound class comprises, for each test sample of the test dataset, or a subset of the test dataset:
- causing one or more loudspeaker of said one or more Man Machine Interface to play the audio frame of the test sample ;
- receiving, through said one or more Man Machine Interface (MMI2), one or more labels belonging to the at least one additional sound class ;
- enriching the test sample with said one or more labels belonging to the at least one additional sound class.

In substance, these steps correspond to letting a user listen to the audio frame of the all or a part of the test samples, and add the labels belonging to the at least one additional sound class to the test samples (in addition to the labels belonging to the initial classes, that were already present). The enrichment can be applied to the whole test dataset, or a subset of the test dataset.

As the test datasets usually has a much smaller size than the sound detection training dataset, the test dataset can be fully completed with manual labels with a limited labelling burden.

It is now referred to figure 5.

Figure 5 is an example of a graphical interface Grlnt5 to allow a labeler to associate sound labels to a sound frame according to an embodiment.

The graphical interface GrInt5 is an example of graphical interface that could be used be the system Sys2 to allow a labeler to enrich a sound detection training dataset with sound detection training samples associating sound labels to audio frame.

To this effect, the labeler can use the button PI5 to play an audio frame, Snd5 in the example of figure 5. Then, the labeler can click to buttons SndLbls representative of sound classes. In the example of figure 5,the labeler can add labels of classes such has "schock", "alarm", "squeak", etc. Some sound classes may comprise sub-classes. For example, the class "Vocalization" has in this example 9 sub-classes. When the labeler clicks on the button "Vocalization", a window SndLblVoc appears, which comprises the sub-classes. The labeler can then provide labels of more precise vocalizations, such as for example "sing", "laugh", "shout", etc. When the labeler has finished to provide the labels, he can click on the button Val5 to validate. The labeler can also click on the button Conf5 to indicate that he is confident with the labeling, so that more weight can be given to this training sample.

When the labeler validates the labeling, a sound training sample can be formed comprising:
- The audio frame Snd5 ;
- The labels provided by the user.

The labels may be typically provided in the expected output format of the sound model to train. For example, if the sound model to train outputs a vector indicating the classes that are detected from an audio frame, such a vector can be formed with the labels provided by the user. For example, if the vector comprises Boolean values, a Boolean values indicating that a sound class is present is added for all the labels that have been entered by the labeler, and a Boolean values indicating that a sound class is absent otherwise. If the vector comprises confidence level of the presence of a sound class, a high confidence value can be added for the classes that have been labeled by the labeler (and even a higher or maximal confidence value if the labeler has indicated through the button Conf5 that he is confident in the labels), and a low or minimal value otherwise. If no sound has been identified be the labeler, each element of the vector will comprise a label representative of the absence of corresponding class. The sound labels for an audio frame could be provided by a single labeler or be a combination of labels provided by a plurality of labelers for a given audio frame.

The figure 5 is provided by means of example only of a graphical interface to provide sound labels according to an embodiment. Other interfaces could be foreseen, provided that they allow a labeler to listen to an audio frame and provide the labels corresponding to the sound classes that have been heard.

It is now referred to figure 6.

Figure 6 is an example of sound classes in an embodiment.

The figure 6 represents an example of an extended set of sound classes. The extended set of sound classes thus corresponds to the sound classes that can be detected, and the labels that can be entered in the training phase.

In an embodiment, the extended set of sound classes comprises at least one super-class that encompasses a plurality of classes of the extended set of sound classes.

The figure 6 represents some of such super-classes, wherein the sound classes encompassed within the super-class are represented under the super-class class. For example, the super-class "squeak" encompasses 3 sub-classes "bed squeak", "door squeak", and "shutter squeak".

This provides the flexibility of labeling the super-class rather than a specific class, for when the detection of a specific class in an audio frame is not possible. For example, only the super-class "squeak" can be labeled if a squeak that is neither a bed, door or shutter squeak is heard in an audio frame.

The set of sound classes represented in figure 6 is adapted to an environment that is a room of a retirement homes and represents various sounds that can be heard in that context. Of course, different sets of classes can be defined according to the environment that is analyzed, and the sets of sound classes would be different in other environments, for example for a playground. It is of course possible to define the most relevant se of classes for a given environment.

It is now described some examples of the sound classes represented in figure 6, and the relevance to detect such types of sounds. The description below is provided by means of non-limitative example only of possible purposes of detecting certain of the sound classes represented in figure 9. The purposes listed below are non-limitative. As it is also apparent, at least from the examples provided in figure 6, many other sound classes could be considered within the present disclosure.

Sighs: Sighs are involuntary respiratory events that can provide valuable insights into an individual's physical and emotional state. In vulnerable populations, such as the elderly or those with chronic conditions, sighs may indicate underlying health issues or emotional distress or discomfort. Frequent sighing may be associated with respiratory problems or other medical conditions. Sighs can be a form of non-verbal communication for those who struggle to express themselves verbally. For vulnerable adults who may have difficulty articulating their needs or feelings, sighs can provide valuable insights into their emotional state. By recognizing and responding to these non-verbal cues, caregivers and professionals can better support vulnerable individuals in making informed choices and expressing their needs. Also, on a pure medical point of view, it has been found that sighing patterns differ between Parkinson Disease and multiple system atrophy (MSA), a related neurodegenerative disorder. Excessive sighing during sleep, particularly in slow-wave sleep, is more characteristic of MSA than PD (see Vargas Gonzalez, E., Yang, Z., Dodet, P. et al. Increased sighing during sleep as a marker of multiple system atrophy. npj Parkinsons Dis. 10, 176 (2024). https://doi.org/10.1038/s41531-024-00765-4 ). Current monitoring systems often overlook these subtle cues, potentially missing early warning signs of deteriorating health or emotional well-being. Detecting sighs thanks to acoustic monitoring can improve the quality of life for vulnerable people.

Aspiration events: Detecting aspiration events in vulnerable individuals, particularly the elderly, holds significant medical importance. Aspiration, often resulting from the accumulation of saliva or other substances (solid or liquid, foods or drugs, pills) in the mouth that are inhaled into the airways, can lead to violent coughing fits. While these episodes are generally benign and resolved quickly, they can become more problematic with age as the individual's ability to clear the airways diminishes. This can result in the accumulation of fluid or other substances in the lungs, increasing the risk of pulmonary infections. Additionally, the aging process affects swallowing quality, further elevating the risk of aspiration events. Repeated aspiration episodes significantly increase the likelihood of pulmonary infections, which can be life-threatening. Monitoring the frequency and severity of aspiration events in vulnerable individuals can provide valuable insights for healthcare providers. This information can aid in anticipating and mitigating the risk of pulmonary infections, as well as enhancing patient care, such as providing nursing assistance during meals to reduce the occurrence of such incidents. By detecting and addressing aspiration events early, healthcare providers can improve the overall well-being and quality of life for vulnerable patients.

Scratching (skin) : In a monitored environment, frequent skin scratching can be easily detected through acoustic monitoring, providing significant benefits for vulnerable individuals. Frequent scratching can indicate underlying dermatological conditions like eczema or psoriasis, the presence of parasites or infections, or even mental health issues such as stress, anxiety, or obsessive-compulsive disorder (OCD). Early detection allows for prompt diagnosis and treatment, preventing skin lesions, secondary infections, and scarring, which can be particularly problematic for elderly or medically fragile individuals. Caregivers receiving alerts thanks to such a system can take immediate actions, such as assessing the individual, applying topical creams, ensuring a clean environment, and providing psychosocial support to address underlying stress or anxiety. Documenting the frequency and severity of scratching episodes aids in long-term monitoring and follow-up, ensuring that persistent issues are addressed effectively. Overall, this approach enhances patient care, prevents complications, and improves the overall well-being of vulnerable individuals.

Urination on the floor : This type of sound is specific and can be easily detected with acoustic monitoring. In specialized facilities, some individuals may experience neurological and cognitive disorders that lead them to urinate voluntarily or involuntarily on the floor. Detecting such events and alerting caregivers can be highly beneficial. Prompt notification allows for immediate cleanup, reducing the risk of falls and injuries from slippery surfaces. Additionally, quick intervention can prevent the spread of bacteria and odors, maintaining a hygienic environment. For individuals with cognitive impairments, frequent urination incidents may indicate underlying medical issues that require attention. Alerting caregivers enables timely assessment and appropriate care, ensuring the safety and well-being of the residents.

Tracheostomy tube obstructions: these events can occur due to various factors, including the accumulation of mucus plugs, blood clots, or thick secretions within the tube. Other causes include the formation of granulomas, improper tube positioning, or pressure from the airway walls. These obstructions can rapidly lead to life-threatening respiratory compromise, making early detection crucial. In a healthcare setting, particularly where resources may be limited, detecting such obstructions is vital for patient safety. Early detection allows for prompt intervention by caregivers, potentially preventing serious complications such as respiratory distress, atelectasis, or lung abscesses. By implementing our alert system based on acoustic monitoring for healthcare providers, obstructions can be addressed quickly through appropriate measures like suctioning, tube repositioning, or replacement, thereby reducing the risk of emergencies and improving overall patient care

It is now referred to figure 7

Figure 7 is a method of detection of sound classes in an audio frame according to an embodiment.

The method P7 may for example be implemented by a computing device such as the device Dev1.1 represented in figure 1.

The method P7 comprises a first step S7.1 of receiving an audio frame from one or more microphone.

In the example of figure 1, the step S7.1 may for example correspond to receiving the audio frames Snd1.1, Snd1.2 from the one or more microphone Mic1.1; Mic1.2 located in the environments Env1.1, Env1.2.

The method P3 further comprises a second step S7.2 of detecting in said audio frame one or more sound classes belonging to an extended set of sound classes, using one or more of:
- a second sound model, such as SndMod2.2, trained according to the method P3 ;
- a first sound model, such as SndMod2.1, re-trained according to the method P4.

As already explained, a sound model is a model that takes as input an audio frames and delivers as output a prediction of sound classes that are present from the audio frame.

The prediction of sound classes may for example take the form of a vector that comprises for each sound class an indication of presence or absence of the sound class (e.g Boolean value, confidence of presence, etc.), if the sound model is a classifier.

The prediction of sound classes may also in another example take the form of a vector that comprises for each sound class a ratio that indicates what would be the ratio of labels representative of presence or absence of the sound class among a plurality of labels received for the audio frame.

Making use of one or more of the second sound model, such as SndMod2.2, trained according to the method P3, and the first sound model, such as SndMod2.1, re-trained according to the method P4 allows benefiting from the predictions of a model that is trained to accurately detect the at least one additional sound classes.

This disclosure is not limited to the method, system devices and computer programs described here, which are only examples. The invention encompasses every alternative that a person skilled in the art would envisage when reading this text.

## Claims

1. A computer-implemented method (P3; P4) comprising:
- obtaining (S3.1) a first trained sound model (SndMod2.1) configured to detect in audio frames one or more sound classes belonging to an initial set of sound classes ;
- receiving (S3.2) from one or more microphones (Mic1.1; Mic1.2) a plurality of audio frames (Snd1.1; Snd1.2);
- for each audio frame of said plurality of audio frames:
∘ causing (S3.3) one or more loudspeaker (Ldspk2) of one or more Man Machine Interface (MMI2) to play said audio frame ;
∘ obtaining (S3.4) one or more labels belonging to an extended set of sound classes comprising the classes of the initial set of sound classes, and at least one additional sound class ;
∘ enriching (S3.5) a sound detection training dataset (SndDS2.2) of a second sound model (SndMod2.2) with said audio frame associated to said one or more labels ;
- using (S3.7) said sound detection training dataset of the second sound model to train said second sound model to detect in audio frames one or more sounds classes belonging to said extended set of sound classes.

2. The method (P4) of claim 1, further comprising:
- retrieving (S4.8) a sound detection training dataset (SndDS2.1) of the first sound model, said sound detection training dataset of the first sound model comprising a plurality of initial samples, each initial sample comprising an audio frame associated with one or more labels belonging to the initial set of sound classes ;
- enriching (S4.9) the sound detection training dataset of the first sound model, with labels belonging to said at least one additional sound class, said enriching comprising, for each initial sample:
o using the second sound model to detect (S4.9.1) in the audio frame of the initial sample the presence or absence of said at least one additional sound class ;
o enriching (S4.9.2) the one or more labels associated to the audio frame of the initial sample with the detection of presence or absence of said at least one additional sound class, so that the audio frame of the initial sample is associated with labels of the extended set of sound classes;
- re-training (S4.10) the first sound model using the enriched sound detection training dataset of the first sound model.

3. The computer-implemented method of claim 2, further comprising:
- retrieving a test dataset of the first sound model, said test dataset comprising a plurality of test samples, each test sample of the test dataset comprising an audio frame associated to one or more labels belonging to the initial set of sound classes ;
- enriching said test dataset with labels belonging to the at least one additional sound class received through said one or more Man Machine Interface (MMI2);
- using said enriched test dataset to verify the training of the re-training of the first sound detection model.

4. The computer-implemented method of claim 3 , wherein enriching said test dataset with labels belonging to the at least one additional sound class comprises, for each test sample of the test dataset, or a subset of the test dataset :
o causing one or more loudspeaker of said one or more Man Machine Interface to play the audio frame of the test sample ;
o receiving, through said one or more Man Machine Interface (MMI2), one or more labels belonging to the at least one additional sound class ;
o enriching the test sample with said one or more labels belonging to the at least one additional sound class.

5. The computer-implemented method of any one of claims 2 to 4, wherein enriching the sound detection training dataset (SndDS2.1) of the first sound model further comprises, adding training samples from the sound detection training dataset (SndDS2.2) of the second sound model to said sound detection training dataset (SndDS2.1) of the first sound model.

6. The computer-implemented method of any one of claims 2 to 5, wherein enriching the sound detection training dataset (SndDS2.1) of the first sound model further comprises:
∘ receiving from said one or more microphone a plurality of subsequent audio frames
∘ for each subsequent audio frame of said plurality of subsequent audio frames:
▪ causing said one or more loudspeaker of said one or more Man Machine Interface (MMI2) to play said subsequent audio frame ;
▪ receiving, through said one or more Man Machine Interface (MMI2), one or more labels belonging to the extended set of sound classes;
▪ enriching the sound detection training dataset (SndDS2.1) of the first sound model (SndMod2.1) with said subsequent audio frame associated to said one or more labels .

7. The computer-implemented method of any of the preceding claims, wherein the first and the second sound models are convolutional neural networks.

8. The computer-implemented method of any of the preceding claims, wherein the first and the second sound models are neural networks having a same architecture.

9. The computer-implemented method of any one of the preceding claims, wherein:
- a loss function used for training at least one of the first sound model and the second sound model comprises a weighted sum of prediction losses of each class of the extended set of sound classes ;
- the weights of the prediction loss of each class of the extended set of sound classes in said weighted sum is a decreasing function of the number of occurrences of the sound classes in the sound detection training dataset of said at least one of the first and the second sound model.

10. The computer-implemented method of any one of the preceding claims, wherein said extended set of sound classes comprises at least one super-class that encompasses a plurality of classes of the extended set of sound classes.

11. A computer-implemented method (P7) comprising
- receiving (S7.1) from one or more microphone (Mic1.1; Mic1.2) an audio frame ;
- detecting (S7.2) in said audio frame one or more sound classes belonging to an extended set of sound classes, using one or more of:
o a second sound model (SndMod2.2) trained using a method according to any one of the preceding claims ;
o a first sound model (SndMod2.1) re-trained using a method according to any one of the claims 2 to 9, when dependent upon claim 2.

12. Computer software comprising instructions to implement at least a part of a method according to any one of the preceding claims when the software is executed by a processor.

13. Computer-readable non-transient recording medium on which a software is registered to implement a method according to one of claims 1 to 11 when the software is executed by a processor.

14. A computer system (Sys2) comprising:
- one or more Man Machine Interface (MMI2) comprising one or more loudspeaker (Ldspk2) ;
- one or more processing unit (Proc2) configured to:
∘ obtain a first trained sound model (SndMod2.1) configured to detect in audio frames one or more sound classes belonging to an initial set of sound classes ;
∘ receive from one or more microphone (Mic1.1; Mic1.2) a plurality of audio frames ;
∘ for each audio frame of said plurality of audio frames:
▪ cause one or more loudspeaker (Ldspk2) of one or more Man Machine Interface (MMI2) to play said audio frame ;
▪ obtain one or more labels belonging to an extended set of sound classes comprising the classes of the initial set of sound classes, and at least one additional sound class ;
▪ enrich a sound detection training dataset (SndDS2.2) of a second sound model (SndMod2.2) with said audio frame associated to said one or more labels ;
∘ use said sound detection training dataset of the second model to train said second sound model to detect in audio frames one or more sounds classes belonging to said extended set.

15. A computing system (Sys1) comprising:
- one or more microphone (Mic1.1; Mic1.2 );
- one or more first computing device (Dev1.1) ;
wherein said one or more first computing device is configured to:
- receive from said one or more microphone an audio frame ;
- detect in said audio frame one or more sound classes belonging to an extended set of sound classes, using one or more of:
o a second model trained using a method according to any one of claims 1 to 8 ;
o a first sound model re-trained using a method according to any one of the claims 2 to 9, when dependent upon claim 2.
